(19) Europäisches Patentamt
European Patent Office
Office européen des brevets

(11) Publication number: **0 612 734 A1**

# (12) EUROPEAN PATENT APPLICATION

(21) Application number: **94102139.6**

(22) Date of filing: **11.02.94**

(51) Int. Cl.[5]: **C07D 249/08**, A61K 31/41, A01N 43/653

(30) Priority: **11.02.93 ES 9300268**

(43) Date of publication of application: **31.08.94 Bulletin 94/35**

(84) Designated Contracting States: **AT BE CH DE DK ES FR GB GR IE IT LI LU MC NL PT SE**

(71) Applicant: **J. URIACH & CIA. S.A.**
**Degà Bahi, 59-67**
**E-08026 Barcelona (ES)**

(72) Inventor: **Bartroli, Javier**
**Cardenal Vives i Tuto 55**
**E-08034-Barcelona (ES)**

Inventor: **Turmo, Enric, Dr.**
**Marti i Julia 112**
**E-08903-L'Hospitalet, Barcelona (ES)**
Inventor: **Carceller, Elena**
**Aragon 117**
**E-08015 Barcelona (ES)**
Inventor: **Almansa, Carmen**
**Independencia 333**
**E-08026 Barcelona (ES)**

(74) Representative: **Zumstein, Fritz, Dr. et al**
**Dr. F. Zumstein**
**Dipl.-Ing. F. Klingseisen**
**Bräuhausstrasse 4**
**D-80331 München (DE)**

(54) **Novel orally active antifungal agents.**

(57) The present invention relates to novel orally active antifungal agents of general formula **I**

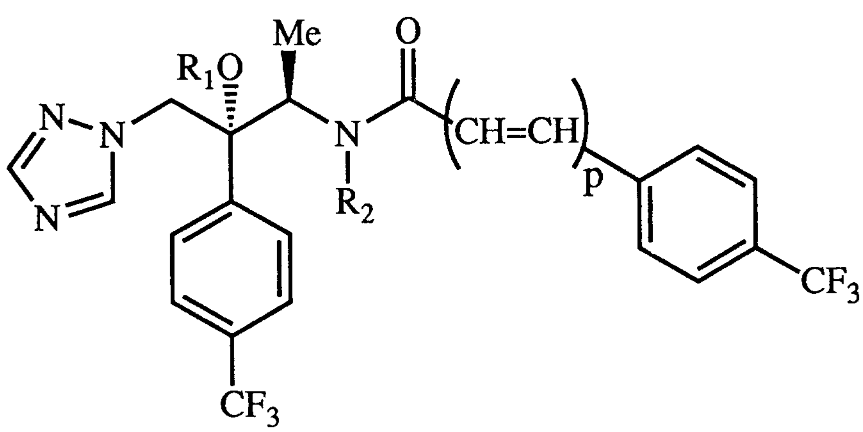

**I**

wherein $R_1$ represents hydrogen and $R_2$ represents hydrogen or $C_{1-4}$ alkyl, or $R_1$ and $R_2$ together represent a group $-(CH_2)-$; p is 0 or 1, and the salts and solvates thereof.

EP 0 612 734 A1

The present invention relates to a new series of trifluoromethylbenzene derivatives of general formula **I** having antifungal activity. The invention also relates to a process for their preparation, to the pharmaceutical compositions containing them and to their use for the manufacture of a medicament for the treatment of fungal diseases.

European patent application EP 332,387, published September 13, 1989 (assigned to Sankyo Company Limited) describes, among others, certain antifungal compounds of general formula

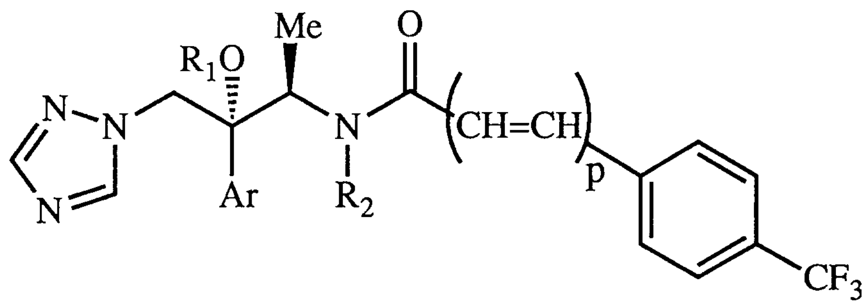

wherein:
Ar represents a phenyl group optionally substituted by one or two halogen or trifluoromethyl groups; $R_1$ is H or together with $R_2$ forms an oxazolidine ring; $R_2$ is H, $C_1$-$C_4$ alkyl or together with $R_1$ forms an oxazolidine ring; and p is 0 or 1. These compounds are useful for the treatment of fungal diseases in animals and plants. The above mentioned patent application discloses as preferred meanings for the susbtituent Ar a phenyl group substituted by one or two halogen atoms, preferably fluorine and/or chlorine. More specifically, EP 332387 mentions as more preferred meanings for Ar the following groups: 4-chlorophenyl, 4-fluorophenyl, 2,4-dichlorophenyl, 2,4-difluorophenyl, 2-chloro-4-fluorophenyl and 2-fluoro-4-chlorophenyl, of which 2,4-dichlorophenyl, 2,4-difluorophenyl, and 4-chlorophenyl are most preferred. Among these compounds, the compound (4R*,5R*)-5-(2,4-difluorophenyl)-4-methyl-5-[(1H-1,2,4-triazol-1-yl)methyl]-3-[4-(trifluoromethyl)benzoyl]oxazolidine (hereinafter referred to as compound **4**) shows an excellent antifungal activity (*Chem. Pharm. Bull.,* **1990**, *38,* 2476). However, this compound has now been found to be toxic at high doses, which severely limits its use in treating human mycoses.

The present invention relates to a series of compounds of general formula **I**

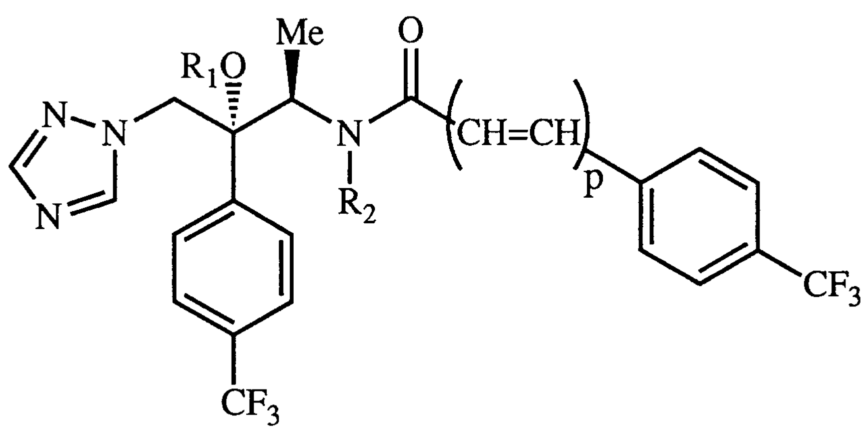

**I**

wherein $R_1$ represents hydrogen and $R_2$ represents hydrogen or $C_{1-4}$ alkyl, or $R_1$ and $R_2$ together represent a group $-(CH_2)-$; p is 0 or 1, and the salts and solvates thereof. This family of compounds is not specifically disclosed in the aforementioned patent application, nor is the 4-(trifluoromethyl)phenyl moiety specifically named as a preferred example of the Ar substituent in their general structural formula. In fact, none of the compounds containing a trifluoromethyl substituent in the Ar ring had actually been prepared. In an exhaustive attempt to reduce the toxicity of this kind of products while retaining their activity, we have

surprisingly found that the substitution of the 2,4-difluorophenyl group by a 4-(trifluoromethyl)phenyl translates into an improvement of the tolerance and toxicity of these products, as shown by the mortality test we have carried out in our laboratory and which is explained in more detail further on. This gives the compounds of the present invention a clear and unexpected advantage over the aforesaid reference compounds in the treatment of fungal infections, a field where compounds must be frequently administered at high doses in order to completely eradicate the infection. The compounds of the present invention represent thus a safer alternative to the above mentioned prior art compounds for use in therapy.

The compounds of general formula **I** are antifungal agents whose mechanism of action is based on the inhibition of the biosynthesis of ergosterol in fungi. Other antifungal agents acting in this way are known in the medical practice and are currently used in therapy. Some of them are applied in the topical treatment of fungal infections of the skin, vagina and nails. More recently discovered compounds are used orally in the treatment of systemic and organ mycoses, such as systemic candidiasis, aspergillosis, criptoccocal meningitis, coccidioidomycosis, paracoccidioidiomycosis, histoplasmosis and blastomycosis. These diseases appear frequently in immunosupressed patients, such as AIDS and cancer patients. Some other compounds related to the ones of the present invention are also used as agrochemicals to protect plants from a variety of fungi.

Accordingly, the present invention also provides the use of a compound of formula **I** or a pharmaceutically acceptable salt or solvate thereof for the manufacture of a medicament for the treatment or prophylaxis of fungal diseases in animals, including man.

The invention further provides a pharmaceutical composition which comprises an effective amount of a compound of formula **I** or a pharmaceutically acceptable salt or solvate thereof in admixture with a pharmaceutically acceptable excipient.

The compounds of the present invention also posess antifungal properties which are useful in combatting a wide variety of plant fungal diseases. The invention thus provides the use of a compound of formula **I** or a salt or solvate thereof for the treatment or prophylaxis of fungal infections in plants. The invention still further provides an agrochemical composition comprising an effective amount of a compound of formula **I** or a salt or solvate thereof in admixture with an agronomically acceptable excipient. The term plant as used herein includes seedlings, bushes and trees.

The invention also provides a process for preparing a compound of formula **I**, which comprises reacting a compound of formula

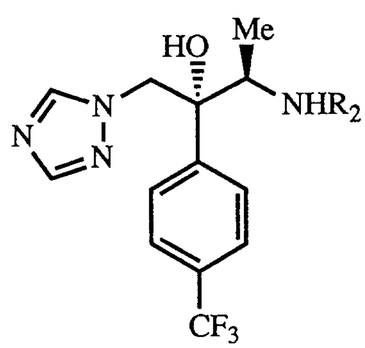

or

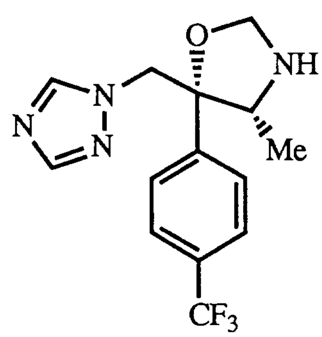

wherein $R_2$ represents hydrogen or $C_{1-4}$ alkyl, with 4-trifluoromethylbenzoic acid or *trans*- 4-trifluoromethylcinnamic acid or a reactive derivative thereof, such as the acid chloride, under standard experimental conditions.

In the compounds of the present invention, a $C_{1-4}$ alkyl group means a linear or branched alkyl chain containing from 1 to 4 carbon atoms, i.e. methyl, ethyl, propyl, isopropyl, *n*-butyl, isobutyl, *sec*-butyl and *tert*-butyl.

Although the present invention encompasses all the compounds described above, compounds wherein $R_1$ represents hydrogen and $R_2$ represents hydrogen, or $R_1$ and $R_2$ together represent a group $-(CH_2)-$ are preferred, i.e.:

- (2R*,3R*)-3-[4-(Trifluoromethyl)benzoylamino]-2-[4-(trifluoromethyl)phenyl]-1-(1H-1,2,4-triazol-1-yl)-2-butanol;
- (2R*,3R*)-3-[*trans*-4-(Trifluoromethyl)cinnamoylamino]-2-[4-(trifluoromethyl)phenyl]-1-(1H-1,2,4-triazol-1-yl)-2-butanol;

- (4R*,5R*)-4-Methyl-5-[(1H-1,2,4-triazol-1-yl)methyl]-3-[4-(trifluoromethyl)benzoyl]-5-[4-(trifluoromethyl)-phenyl]oxazolidine;
- (4R*,5R*)-4-Methyl-5-[(1H-1,2,4-triazol-1-yl)methyl]-3-[*trans*-4-(trifluoromethyl)cinnamoyl]-5-[4-(trifluoromethyl)phenyl]oxazolidine.

The compounds of formula **I** contain a basic nitrogen atom and, consequently, they can form salts, which are also included in the present invention. There is no limitation on the nature of these salts, provided that, when used for therapeutic purposes, they are pharmaceutically acceptable, which, as is well-known in the art, means that they do not have reduced activity (or unacceptable reduced activity) or increased toxicity (or unacceptable increased toxicity) compared with the free compounds. Examples of these salts include: salts with an inorganic acid such as hydrochloric acid, hydrobromic acid, hydriodic acid, nitric acid, perchloric acid, sulfuric acid or phosphoric acid; and salts with an organic acid, such as methanesulfonic acid, trifluoromethanesulfonic acid, ethanesulfonic acid, benzenesulfonic acid, *p*-toluenesulfonic acid, fumaric acid, oxalic acid or maleic acid.

The compounds of formula **I** contain two asymmetric centers and, therefore, they can exist as two pairs of enantiomers. The present invention relates to the racemic compounds of relative stereochemistry (R*,R*) and more particularly to their biologically active enantiomers, whose absolute configuration is (R,R).

The invention also provides a process for the preparation of the compounds of formula **I**, which is shown in scheme 1:

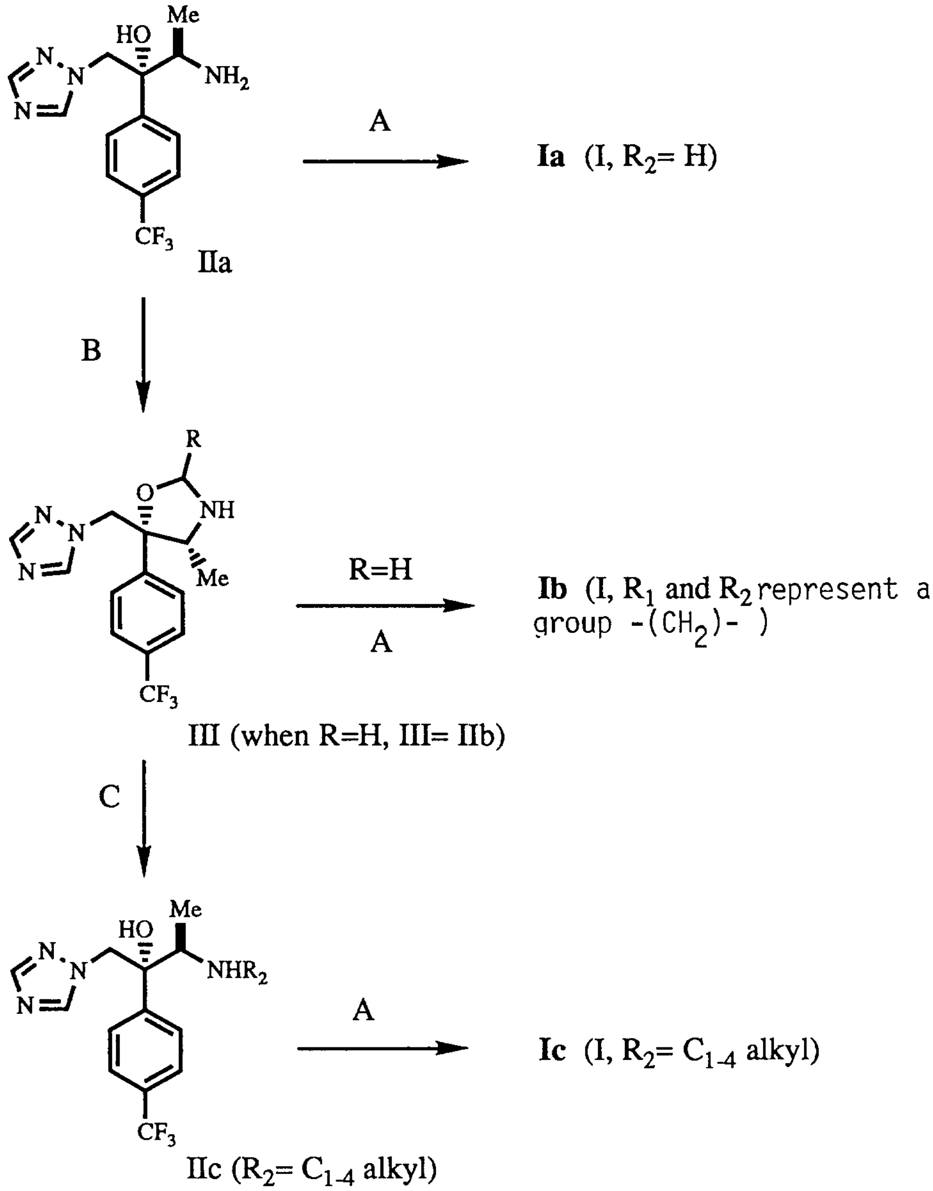


Scheme 1

wherein R represents hydrogen or $C_{1-3}$ alkyl.

Compounds of formula **I** are prepared from amines of formula **IIa**, **IIb** or **IIc** by reaction with 4-trifluoromethylbenzoic acid chloride or 4-trifluoromethylcinnamic acid chloride (step A) in the presence of a proton scavenger base, such as triethylamine or pyridine, in a suitable solvent, such as chloroform or dichloromethane, or the same proton scavenger base can be used as solvent. The reaction is carried out at a temperature between -10 °C and that of the boiling point of the solvent, preferably between 0 and 25 °C, during a period of time from 30 min to 24 h. If required, these compounds can be purified by conventional methods such as flash chromatography or recrystallization. As an alternative to the acid chloride, the anhydride can be employed. Alternatively, compounds of formula **I** may be prepared by a dehydration procedure between amines **IIa**, **IIb** or **IIc** and 4-trifluoromethylbenzoic acid or 4-trifluoromethylcinnamic acid. This process can be carried out using any conventional reaction of amide bond formation, such as reacting an amine with an acid in the presence of dicyclohexylcarbodiimide and 1-hydroxybenzotriazole in a suitable solvent like dioxane, tetrahydrofuran, acetonitrile, chloroform or N,N-dimethylformamide, at a temperature between 0 °C and that of the boiling point of the solvent.

Starting compounds of formula **IIb** and **IIc** are prepared from amine **IIa** as shown in scheme 1 above. In a first step (step B), amine **IIa** is reacted with a compound of formula RCHO, wherein R means hydrogen or $C_{1-3}$ alkyl, in a suitable solvent such as an aromatic hydrocarbon, for example benzene or toluene, at a

temperature from 50 °C to that of the boiling point of the solvent to give the corresponding oxazolidine **III**. When R = H, the compound **III** obtained is starting amine of formula **IIb**. The compounds of formula **IIc** - (wherein $R_2$ represents $C_{1-4}$ alkyl) can be prepared by reduction of the corresponding oxazolidine **III**, wherein R = H or $C_{1-3}$ alkyl, for example with $LiAlH_4$ in an inert solvent such as an ether like diethyl ether, tetrahydrofuran or dioxane, at a temperature between -20 °C and that of the boiling point of the solvent.

The preparation of the compound of formula **IIa** starts from 4-trifluoromethylbenzaldehyde and is outlined in scheme 2:

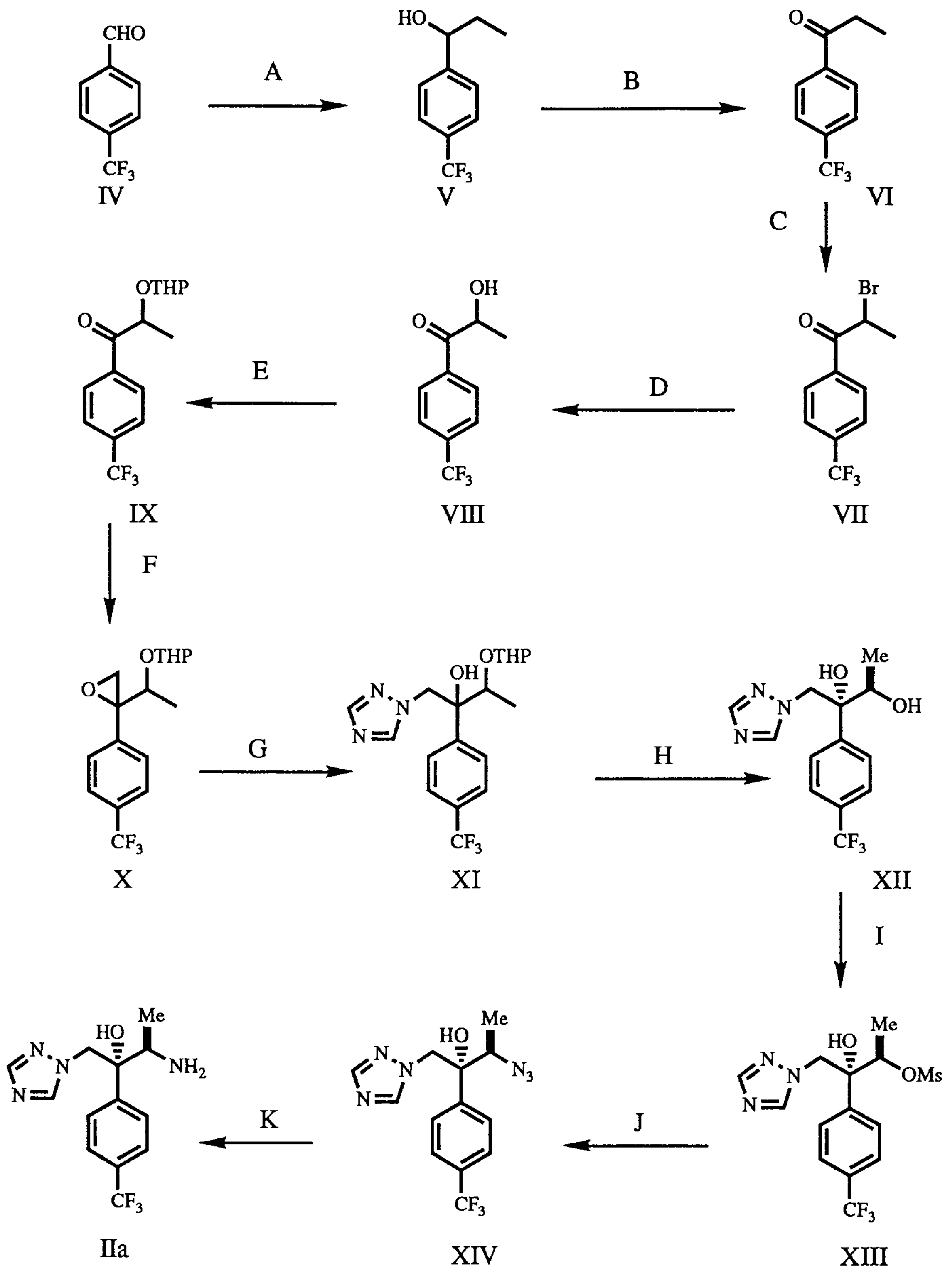


IV V VI VII VIII IX X XI XII XIII XIV IIa

Scheme 2

In a first step (step A) commercially available 4-trifluoromethylbenzaldehyde(**IV**) is allowed to react with ethylmagnesium bromide in an inert solvent, such as tetrahydrofuran, at a temperature between 0 °C and that of the boiling point of the solvent. The corresponding alcohol (**V**) is then oxidized by treatment for example with pyridinium chlorochromate (PCC) (step B) in the presence of celite in a suitable solvent, such as chloroform or dichloromethane, at room temperature to give 4-trifluoromethylpropiophenone (**VI**). This

product is then converted to the α-bromo derivative **VII** by treatment for example with a solution of $Br_2$ in acetic acid (step C) at a temperature between room temperature and 50 °C. The product obtained is then converted to the α-hydroxy derivative **VIII**, for example by treatment with lithium hydroxide (step D) in a suitable solvent, such as a mixture of dimethylformamide and water, at a temperature between 0 °C and room temperature. At this point it may be convenient to purify the intermediate **VIII** for example by chromatography on silica gel. Next, the hydroxy group of the compound **VIII** is protected, for example as the tetrahydropyranyl ether (step E) by treatment with 2,3-dihydropyran and a catalytic amount of an organic acid, such as pyridinium *p*-toluenesulfonate, in a halogenated solvent, such as dichloromethane, to give a compound of formula **IX**. This product is then converted to the corresponding epoxide (**X**) (step F) by treatment for example with trimethylsulfoxonium iodide in a suitable solvent such as dimethylsulfoxide. Then, the epoxide **X** is treated with potassium tertbutoxide and triazole in a suitable solvent such as dimethylformamide at a temperature between room temperature and that of the boiling point of the solvent (step G), to give a compound of formula **XI**, which is then deprotected to give a diol of formula **XII**. The reagent and the reaction conditions needed will depend on the nature of the protecting group used. If the hydroxy group is protected as the tetrahydropyranyl ether, deprotection can be carried out by treatment with *p*-toluenesulfonic acid in a suitable solvent such as methanol at room temperature (step H). The diastereosiomeric mixture of diols (**XII**) thus obtained is purified by recristallization in a suitable solvent like ethyl acetate. The minor isomer (2R*,3S*) remains in the solution and the major isomer (2R*,3R*), which is the desired product, precipitates in pure form. In the next step (step I), diol **XII** is selectively mesylated on the secondary hydroxy group by treatment with methanesulfonyl chloride in a suitable solvent such as pyridine at a temperature between 0 °C and room temperature, to give a compound of formula **XIII**, which is then converted to the azido compound **XIV** (step J) by reaction with sodium azide in the presence of an ammonium halide, preferably ammonium chloride, in a suitable solvent such as dimethylformamide at a temperature between room temperature and that of the boiling point of the solvent, more preferably at a temperature between 100 and 140 °C. Finally, compound **XIV** is converted to the amine **IIa** (step K) by hydrogenation in the presence of a catalyst, such as palladium, in a suitable solvent such as an alcohol, for example ethanol, at a pressure between 1 and 5 atm. The reaction can take place over a wide range of temperatures and the precise reaction temperature is not critical to the invention. In general, we find it convenient to carry out the reaction at about room temperature.

According to the activity of the compounds disclosed, the present invention further provides compositions that contain one or more compounds of the present invention, together with an excipient and optionally other auxiliary agents, if necessary. The compounds of the present invention can be administered in different pharmaceutical preparations, the precise nature of which will depend, as it is well known, upon the chosen route of administration and the nature of the pathology to be treated.

Thus, solid compositions according to the present invention for oral administration include compressed tablets, dispersible powders, granules and capsules. In tablets, one or more of the active component(s) is admixed with at least one inert diluent such as lactose, starch, mannitol, microcrystalline cellulose or calcium phosphate; granulating and disintegrating agents for example corn starch, gelatine, microcrystalline cellulose or polyvinylpyrrolidone; and lubricating agents for example magnesium stearate, stearic acid or talc. The tablets may be coated by known techniques to delay disintegration and absorption in the gastrointestinal tract and, thereby, provide a sustained action over a longer period. Gastric film-coated or enteric film-coated can be made with sugar, gelatin, hydroxypropylcellulose, or acrylic resins. Tablets with a sustained action may also be obtained using an excipient which provides regressive osmosis, such as the galacturonic acid polymers. Formulations for oral use may also be presented as hard capsules of absorbable material, such as gelatin, wherein the active ingredient is mixed with an inert solid diluent and lubricating agents, or pasty materials, such as ethoxylated saturated glycerides. Soft gelatin capsules are possible wherein the active ingredient is mixed with water or an oil medium, for example peanut oil, liquid paraffin or olive oil.

Dispersible powders and granules suitable for preparation of a suspension by the addition of water provide the active ingredient in admixture with a dispersing or wetting agent, a suspending agent, such as sodium carboxymethylcellulose, sodium alginate, polyvinylpirrolidone, gum tragacanth, xantham gum, gum acacia, and one or more preservatives, such as methyl or *n*-propyl-*p*-hydroxybenzoate. Additional excipients, for example sweetening, flavoring and coloring agents may also be present.

Liquid compositions for oral administration include emulsions, solutions, suspensions, syrups and elixirs containing commonly used inert diluents, such as distilled water, ethanol, sorbitol, glycerol, or propylene glycol. Such compositions may also comprise adjuvants such as wetting agents, suspending agents, sweetening, flavoring, perfuming, preserving agents and buffers.

Other compositions for oral administration include spray compositions, which may be prepared by known methods and which comprise one or more active compound(s). The spray compositions will contain a suitable propellent.

Preparations for injection according to the present invention for parenteral administration include sterile aqueous or non-aqueous solutions, suspensions or emulsions, in a non-toxic parentally-acceptable diluent or solvent. Examples of aqueous solvents or suspending media are distilled water for injection, the Ringer's solution, and isotonic sodium chloride solution. Examples of non-aqueous solvents or suspending media are propylene glycol, polyethylene glycol, vegetable oils such as olive oil, or alcohols such as ethanol. These compositions may also include adjuvants such as wetting, preserving, emulsifying and dispersing agents. They may be sterilized by one of the known methods or manufactured in the form of sterile solid compositions which can be dissolved in sterile water or some other sterile injectable medium immediately before use. When all of the components are sterile, the injectables will maintain the sterility if they are manufactured in sterile environment.

A compound of the invention may also administered in the form of suppositories for rectal administration of the drug, or as creams, ointments, pastes, lotions, gels, sprays, foams, aerosols, solutions, suspensions or powders for topical use and as vaginal ovules, tablets, capsules, creams and vaginal douches for vaginal administration. Such compositions are prepared following conventional procedures well known to those skilled in the art.

The dosage and frequency of dose may vary depending upon the nature of the fungal disease, symptoms, age and body weight of the patient, as well as upon the route of administration. Thus, for oral and parenteral administration to human patients the daily dose will be from 1 to 1000 mg for an adult, preferably a dosage of from 5 to 500 mg, which may be administered either as a single dose or as divided doses. For topical administration, a cream or ointment containing 1-10% of a compound of formula **I** will be applied to the skin from one to three times daily.

Following are some representative preparations for tablets, capsules, aerosols and injectables. They can be prepared following standard procedures and they are useful in the treatment of fungal diseases.

| **Tablets** | |
|---|---|
| Compound of formula **I** | 100 mg |
| Dibasic calcium phosphate | 125 mg |
| Sodium starch glycolate | 10 mg |
| Talc | 12.5 mg |
| Magnesium stearate | 2.5 mg |
| | 250.0 mg |

| **Hard gelatin capsules** | |
|---|---|
| Compound of formula **I** | 100 mg |
| Lactose | 197 mg |
| Magnesium stearate | 3 mg |
| | 300 mg |

| **Aerosol** | |
|---|---|
| Compound of formula **I** | 4 g |
| Flavouring agent | 0.2 g |
| Propylene glycol to | 100 ml |
| Suitable propellent to | 1 unit |

| Injectable preparation | |
|---|---|
| Compound of formula **I** | 100 mg |
| Benzylic alcohol | 0.05 ml |
| Propylene glycol | 1 ml |
| Water to | 5 ml |

The following examples illustrate, but do not limit, the scope of the present invention:

**REFERENCE EXAMPLE 1**

**1-[4-(Trifluoromethyl)phenyl]-1-propanol**

Dry tetrahydrofuran (400 mL), magnesium turnings (6.7 g, 0.28 mol) and a iodine crystal were placed in a flask and the mixture was stirred intensively. Ethyl bromide was added dropwise and the resulting mixture was allowed to stir for 30 min. Then, the reaction was cooled to 0 °C and 4-trifluoromethylbenzaldehyde was added dropwise. After stirring at room temperature for 2 h, the reaction mixture was cooled to 0 °C and 1N HCl (250 mL) was added carefully. The mixture was concentrated and the aqueous residue extracted with dichloromethane. The organic layer was dried and the solvent evaporated to afford the title compound of the example as a brown oil (45.2g).
$^1$H RMN (80 MHz, $CDCl_3$) $\delta$ (TMS): 7.52 (q, J = 5.7 Hz, 4H, arom), 1.73 (q, J = 6.8 Hz, 2H, $CH_2$), 0.92 (t, J = 6.8 Hz, 3H, Me).

**REFERENCE EXAMPLE 2**

**4-Trifluoromethylpropiophenone**

A solution of the compound obtained in reference example 1 (23 g, 0.11 mol) in dichloromethane (60 mL) was added dropwise to a suspension of pyridiniumchlorochromate (PCC) (36.6 g, 0.17 mol) and celite (35 g) in dichloromethane (260 mL) at room temperature. The mixture was stirred for 1 h and then diethyl ether (500 mL) was added. The resulting mixture was filtered through celite, washed with aqueous NaOH solution, dried and concentrated. The title compound of the example was obtained as a dark brown oil (20.7 g).
$^1$H RMN (80 MHz, $CDCl_3$) $\delta$ (TMS): 8.05 (d, J = 8.2 Hz, 2H, arom), 7.71 (d, J = 8.2 Hz, 2H, arom), 3.04 (q, J = 7.1 Hz, 2H, $CH_2$), 1.24 (t, J = 7.1 Hz, 3H, Me).

**REFERENCE EXAMPLE 3**

**$\alpha$-Bromo-4-trifluoromethylpropiophenone**

To a solution of the compound obtained in reference example 2 (37 g, 0.18 mol) in acetic acid (600 mL) was added dropwise a solution of 5% $Br_2$ in acetic acid (190 mL) at room temperature. When the addition was complete, the reaction mixture was stirred at 40 °C for 2.5 h. Then, acetic acid was distilled off and the residue was diluted with ethyl acetate (300 mL) and washed with 10% aqueous $NaHCO_3$ solution. The organic phase was dried and the solvent was removed to afford the title compound of the example as an oil (46.9 g).
$^1$H RMN (80 MHz, $CDCl_3$) $\delta$ (TMS): 8.13 (d, J = 8.2Hz, 2H, arom), 7.74 (d, J = 8.2 Hz, 2H, arom), 5.26 (q, J = 6.6 Hz, 1H, CHBr), 1.92 (d, J = 6.6 Hz, 3H, Me).

**REFERENCE EXAMPLE 4**

**$\alpha$-Hydroxy-4-trifluoromethylpropiophenone**

To a solution of the compound obtained in reference example 3 (45.9 g, 0.16 mol) in a 4:1 mixture of DMF and $H_2O$ (460 mL) was added $LiOH.H_2O$ (6.9 g, 0.16 mol) at 0 °C and the resulting mixture was stirred for 2 h. After diluting with ethyl acetate, the mixture was washed with brine to afford 39.81 g of a yellowish oil. Purification by chromatography on silica gel (eluent: hexaneethyl acetate mixtures of increasing polarity) yielded 23.46 g of the desired compound.

$^1H$ RMN (80 MHz, $CDCl_3$) δ (TMS): 8.05 (d, J = 8.2Hz, 2H, arom), 7.74 (d, J = 8.2 Hz, 2H, arom), 5.2 (Br q, 1H, CH), 1.45 (d, J = 7Hz, 3H, Me).

**REFERENCE EXAMPLE 5**

**α-(Tetrahydropyran-2-yloxy)-4-trifluoromethylpropiophenone**

A mixture containing the compound obtained in reference example 4 (23.46 g, 0.11 mol), 2,3-dihydropyran (12.6 mL, 0.14 mol), pyridinium *p*-toluenesulfonate (0.006 mol) and dichloromethane (260 mL) was stirred at room temperature for 24 h. Then, 10% aqueous $NaHCO_3$ solution was added and the layers were separated. The organic layer was washed with brine and dried, and the solvent was evaporated to afford the desired product (28.6 g).
$^1H$ RMN (80 MHz, $CDCl_3$) δ (TMS): 8.2-7.9 (m, 2H, arom), 7.71 (d, J = 8.2 Hz, 2H, arom), 5.3-4.5 (m, 2H), 4.2-3.3 (m, 2H), 2.0-1.3 (m, 9H).

**REFERENCE EXAMPLE 6**

**2-[1-(Tetrahydropyran-2-yloxy)ethyl]-2-[4-(trifluoromethyl)phenyl]oxirane**

A suspension of 55% NaH (2.7 g, 0.11 mol) was added to anhydrous DMSO (300 mL) and the mixture was heated at 60 °C for 1.5 h. The reaction mixture was allowed to cool to room temperature and trimethylsulfoxonium iodide (41.6 g, 0.19 mol) was added, after which the mixture was allowed to react for 1 h. Then the compound obtained in reference example 5 (28.6 g, 0.09 mol) in DMSO (200 mL) was added. After stirring for 2 h, the mixture was partitioned between benzene and water. The organic layer was separated and dried, and the solvent evaporated to afford a crude product as a brown oil (29.7 g).
$^1H$ RMN (80 MHz, $CDCl_3$) δ (TMS): 8.60 (s, 4H, arom), 5.4-4.6 (m, 1H, OCHO), 4.4-3.2 (m, 3H), 3.1 (m, 1H, epoxide), 2.7 (m, 1H, epoxide), 1.8-1.4 (m, 6H), 1.4-1.0 (m, 3H, Me).

**REFERENCE EXAMPLE 7**

**(2R*,3R*)-1-(1-H-1,2,4-Triazol-1-yl)-2-[4-(trifluoromethyl)phenyl]-2,3-butanediol**

The crude product obtained in reference example 6 was dissolved in DMF (300 mL) and then treated with 1,2,4-triazole (26.0 g, 0.37 mol) and potassium tert-butoxide (21.12 g, 0.19 mol) at 100 °C for 1 h. DMF was distilled off and the residue was partitioned between benzene and water. The organic phase was separated, washed with brine, dried and concentrated to afford a brown oil (36.28 g). This product was then dissolved in methanol (300 mL) and treated with *p*-toluenesulfonic acid (17.9 g, 0.094 mol) at room temperature for 2 h. 10% $NaHCO_3$ solution was added and methanol was evaporated. The residue was extracted with ethyl acetate, dried and concentrated to afford a brown solid. Recrystallization from ethyl acetate yielded the pure product as a white solid (8.41 g).
m.p.: 177-178 °C;
$^1H$ RMN (80 MHz, MeOH-$d_4$ + $CDCl_3$) δ (TMS): 7.98 (s, 1H, CH triazole), 7.73 (s, 1H, CH triazole), 7.6-7.3 (m, 4H, arom), 4.72 (s, 1H, $CH_2$), 4.16 (q, $J_q$ = 7.0Hz, 1H, C$\underline{H}$Me), 0.96 (d, J = 7Hz, 3H, CH$\underline{Me}$).
Analysis Calcd. for $C_{13}H_{14}F_3N_3O_2$: C 51.83 H 4.68; N 13.95. Found: C 51.96; H 4.63; N 13.61.

**REFERENCE EXAMPLE 8**

**(2R*,3R*)-3-Methanesulfonyloxy-2-[4-(trifluoromethyl)phenyl]-1-(1-H-1,2,4-triazol-1-yl)-2-butanol**

To a cooled solution (0 °C) of the compound obtained in reference example 7 (8.4 g, 0.028 mol) in pyridine (300 mL) was added methanesulfonyl chloride (5.11 g, 0.045 mol) and the reaction mixture was stirred at room temperature for 2 h. Then pyridine was distilled off, and the residue was partitioned between dichloromethane and 10% aqueous $NaHCO_3$ solution The organic phase was separated, dried and concentrated to afford the desired product (9.37 g).
$^1H$ RMN (80 MHz, MeOH-$d_4$ + $CDCl_3$) δ (TMS): 8.00 (s, 1H, CH triazole), 7.72 (s, 1H, CH triazole), 7.54 (s, 4H, arom), 5.16 (q, $J_q$ = 7Hz, 1H, C$\underline{H}$Me), 4.77 (s, 1H, $CH_2$), 3.16 (s, 3H, $MeSO_2$), 1.24 (d, J = 7Hz, 3H, CH$\underline{Me}$).

**REFERENCE EXAMPLE 9**

**(2R*,3R*)-3-Azido-1-(1-H-1,2,4-triazol-1-yl)-2-[4-(trifluoromethyl)phenyl]-2-butanol**

A solution of the compound obtained in reference example 8 (9.4 g, 0.025 mol), sodium azide (8.4 g, 0.13 mol) and ammonium chloride (1.3 g, 0.025 mol) in DMF (140 mL) was heated at 115 °C for 15 h. DMF was distilled off, and the residue was partitioned between water and benzene. The organic layer was washed with brine, dried and concentrated to afford the title compound of the example as a yellowish oil (8.7 g).

**REFERENCE EXAMPLE 10**

**(2R*,3R*)-3-Amino-1-(1-H-1,2,4-triazol-1-yl)-2-[4-(trifluoromethyl)phenyl]-2-butanol**

A suspension of the compound obtained in reference example 9 (0.5 g, 1.5 mmol) and 10% Pd/C (0.14 g) in ethanol (15 mL) was hydrogenated ($H_2$, 1 atm) at room temperature for 1 h with intensive stirring. The resulting mixture was filtered through celite and concentrated to afford the corresponding amine as a white solid (0.46 g).
$^1$H RMN (80 MHz, MeOH-$d_4$ + $CDCl_3$) δ (TMS): 7.88 (s, 1H, CH triazole), 7.86 (s, 1H, CH triazole), 7.47 (q, J = 8 Hz, 4H, arom), 4.53 (s, 2H, $CH_2$), 3.38 (q, $J_q$ = 7Hz, 1H, C<u>H</u>Me), 0.85 (d, J = 7Hz, 3H, CH<u>Me</u>).

**REFERENCE EXAMPLE 11**

**(4R*,5R*)-4-Methyl-5-[(1-H-1,2,4-triazol-1-yl)methyl]-5-[4-(trifluoromethyl)phenyl] oxazolidine**

A solution of the compound obtained in reference example 10 (250 mg, 0.83 mmol) and *p*-formaldehyde (25 mg, 0.84 mmol) in benzene was heated at reflux for 3 h. The resulting mixture was concentrated to afford the desired product as an oil.
$^1$H RMN (80 MHz, $CDCl_3$) δ (TMS): 8.18 (s), 7.86 (s), 7.68 (s), 7.57 (s), 7.46(s), 7.35 (s), 4.83 (d, J = 6.3Hz, 1H, C<u>H</u>(H)), 4.67 (s, 2H, CH2), 4.33 (d, J = 6.3Hz, 1H, CH(<u>H</u>)), 3.40 (q, J = 7.0Hz C<u>H</u>Me), 0.89 (d, J = 7Hz, CH<u>Me</u>, 3H).

**EXAMPLE 1**

**(2R*,3R*)-3-[4-(Trifluoromethyl)benzoylamino]-2-[4-(trifluoromethyl)phenyl]-1-(1H-1,2,4-triazol-1-yl)-2-butanol (1)**

To a solution of (2R*,3R*)-3-amino-1-(1-H-1,2,4-triazol-1-yl)-2-[4-(trifluoromethyl)phenyl]-2-butanol (0.5 g, 1.6 mmol) (obtained according to the procedure described in reference example 10) in dichloromethane (15 mL), was added triethylamine (0.2 mL, 2 mmol). After cooling the resulting mixture in an ice bath, a solution of 4-(trifluoromethyl)benzoyl chloride (0.38 g, 1.8 mmol) in dichloromethane (5 mL) was added and the reaction mixture was stirred for 2 h at 0 °C. The resulting mixture was diluted with $CHCl_3$ and washed with 5% aqueous $NaHCO_3$. The organic phase was separated, dried over sodium sulfate and the solvent was removed to afford a thick oil. Purification by chromatography on silica gel (ethyl acetate-hexane, 1:1) yielded the compound **1** as a white powder. This solid was recrystallized from ethyl acetate-ether to give 0.481 g of a white crystalline solid.
m.p.: 198-199 °C;
$^1$H RMN (80 MHz, $CDCl_3$) δ (TMS): 8.02 (s), 7.92 (s), 7.81 (s), 7.69 (s), 7.63 (s), 7.52 (s), 6.76 (br d, J = 10 Hz, NH), 4.73 (d, J = 15Hz, 1H, C<u>H</u>(H)), 4.49 (d, J = 15Hz, 1H, CH(<u>H</u>)), 3.48 (q, J = 7.0Hz, C<u>H</u>Me), 1.24 y 1.04 (d, J = 7Hz, CH<u>Me</u>, 3H).
Analysis Calcd. for $C_{21}H_{18}F_6N_4O_2$: C 53.39; H 3.84; N 11.86. Found: C 52.96; H 4.03; N 11.53.

**EXAMPLE 2**

**(4R*,5R*)-4-Methyl-5-[(1H-1,2,4-triazol-1-yl)methyl]-3-[4-(trifluoromethyl)benzoyl]-5-[4-(trifluoromethyl)phenyl]oxazolidine (2)**

Following the procedure described in example 1, but starting from the compound obtained in reference example 11, the title compound of this example was obtained as a white solid.
m.p.: 182-183 °C;
$^1$H RMN (80 MHz, $CDCl_3$) δ (TMS): 7.85 (s, 1H, triazole), 7.73 (s, 8H, arom), 7.58 (s, 1H, triazole), 5.3 (br d, 2H, $NCH_2O$), 5.1 (br q, 1H, C$\underline{H}$Me), 4.56 (s, 2H, $CH_2$), 0.90 (d, J = 7Hz CH$\underline{Me}$, 3H).
Analysis Calcd. for $C_{22}H_{18}F_6N_4O_2$: C 54.55; H 3.75; N 11.57. Found: C 54.86; H 3.83; N 11.33.

**EXAMPLE 3**

**(4R*,5R*)-4-Methyl-5-[(1H-1,2,4-triazol-1-yl)methyl]-3-[*trans*-4-(trifluoromethyl) cinnamoyl]-5-[4-(trifluoromethyl)phenyl]oxazolidine (3)**

Following the procedure described in example 2, but using *trans*-4-(trifluoromethyl)cinnamic acid chloride instead of 4-(trifluoromethyl)benzoic acid chloride, the title compound of the example was obtained as a white solid. m.p.: 157-158 °C;
$^1$H RMN (80 MHz, $CDCl_3$) δ (TMS): 7.86 (d, J = 7.2 Hz, 1H, CH = ), 7.8-7.3 (m, 11H, arom, triazole, CH = ), 5.7-5.3 (m, 2H, $NCH_2O$), 4.98 (q, J = 6.5 Hz, 1H, C$\underline{H}$Me), 4.58 (br s, 2H, $CH_2$), 0.95 (d, J = 6.5Hz, CH$\underline{Me}$, 3H).
Analysis Calcd. for $C_{24}H_{20}F_6N_4O_2$: C 56.47; H 3.95; N 10.98. Found: C 56.32; H 3.97; N 10.84.

**EXAMPLE 4**

**In vivo testing**

The in vivo evaluation was carried out using an *in vivo* model of systemic candidiasis in mice (as described in EP 332,387). According to this model wherein all untreated animals died between days 2 and 3 after infection, animals treated with the compounds of examples 1, 2 and 3 administered at a dose of 20 mg/kg p.o. at 1, 4 and 24 h post infection showed a 100% survival rate at the end of the study (day 10).

**EXAMPLE 5**

**Toxicity testing**

A comparative study of the toxicity of the compound (4R*,5R*)-4-methyl-5-[(1H-1,2,4-triazol-1-yl)methyl]-3-[4-(trifluoromethyl)benzoyl]-5-[4-(trifluoromethyl)phenyl]oxazolidine (**2**) (prepared in example 2) and reference compound (4R*,5R*)-5-(2,4-difluorophenyl)-4-methyl-5-[(1H-1,2,4-triazol-1-yl)methyl]-3-[4-(trifluoromethyl)benzoyl]oxazolidine (**4**) was carried out. Test compounds were administered every day for 5 days at a dose of 1000 mg/kg/day to groups of 5 male mice and the mortality during the treatment period was recorded. All mice treated with compound **4** died within the first day of treatment whereas no death was recorded during the 5-day period in the group of animals treated with compound **2**.

**Claims**

**1.** A compound of formula **I**

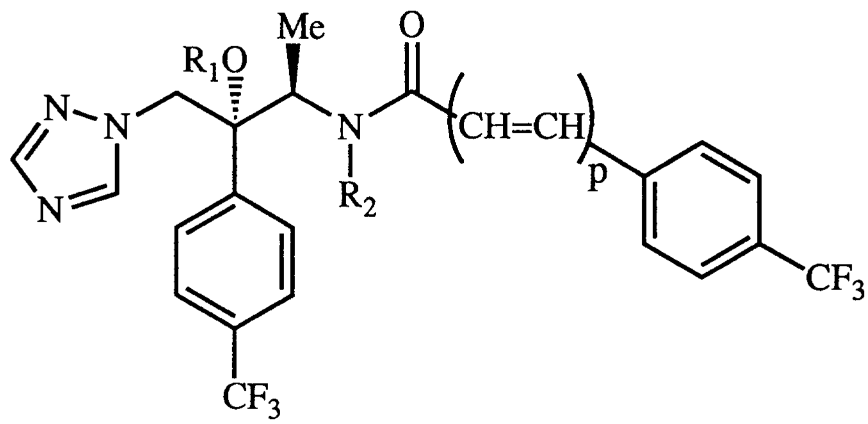

I

as the racemate (R*,R*) or the pure biologically active enantiomer (R,R), wherein:

$R_1$ represents hydrogen;

$R_2$ represents hydrogen or $C_1$-$C_4$ alkyl;

or $R^1$ and $R^2$ together represent a group -($CH_2$)-

p is 0 or 1;

and the salts and solvates thereof.

**2.** (2R*,3R*)-3-[4-(Trifluoromethyl)benzoylamino]-2-[4-(trifluoromethyl)phenyl]-1-(1H-1,2,4-triazol-1-yl)-2-butanol and the salts and solvates thereof.

**3.** (2R*,3R*)-3-[*trans*-4-(Trifluoromethyl)cinnamoylamino]-2-[4-(trifluoromethyl)phenyl]-1-(1H-1,2,4-triazol-1-yl)-2-butanol and the salts and solvates thereof.

**4.** (4R*,5R*)-4-Methyl-5-[(1H-1,2,4-triazol-1-yl)methyl]-3-[4-(trifluoromethyl)benzoyl]-5-[4-(trifluoromethyl)-phenyl]oxazolidine and the salts and solvates thereof.

**5.** (4R*,5R*)-4-Methyl-5-[(1H-1,2,4-triazol-1-yl)methyl]-3-[*trans*-4-(trifluoromethyl)cinnamoyl]-5-[4-(trifluoromethyl)phenyl]oxazolidine and the salts and solvates thereof.

**6.** A process for preparing a compound of formula **I** as defined in claim 1 which comprises reacting a compound of formula

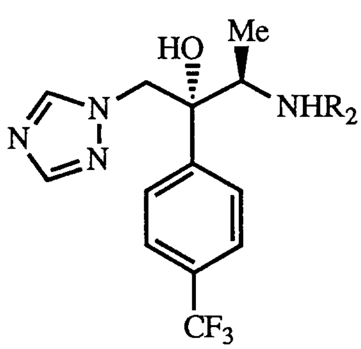

or

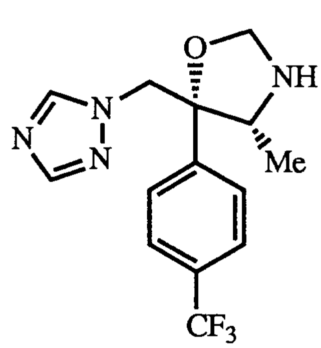

wherein $R_2$ represents hydrogen or $C_{1-4}$ alkyl, with 4-trifluoromethylbenzoic acid or *trans*- 4-trifluoromethylcinnamic acid or a reactive derivative thereof, such as the acid chloride, under standard experimental conditions.

7. A pharmaceutical composition which comprises an effective amount of a compound of formula **I** as defined in claim 1 or a pharmaceutically acceptable salt or solvate thereof in admixture with a pharmaceutically acceptable excipient.

8. The use of a compound of formula **I** as defined in claim 1 or a pharmaceutically acceptable salt or solvate thereof for the manufacture of a medicament for the treatment or prophylaxis of fungal diseases in animals, including man.

9. An agrochemical composition comprising an effective amount of a compound of formula **I** as defined in claim 1 or a salt or solvate thereof in admixture with an agronomically acceptable excipient.

10. The use of a compound of formula **I** as defined in claim 1 for the treatment or prophylaxis of fungal infections in plants.

European Patent Office

# EUROPEAN SEARCH REPORT

Application Number
EP 94 10 2139

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| D,X | EP-A-0 332 387 (SANKYO COMPANY LTD.)<br>* claims * | 1-10 | C07D249/08<br>A61K31/41<br>A01N43/653 |
| A | EP-A-0 435 081 (SS PHARMACEUTICAL CO., LTD.)<br>* claims * | 1-10 | |
| A | EP-A-0 480 215 (J. URIACH & CIA. S.A.)<br>* claims * | 1-10 | |
| A | EP-A-0 145 314 (IMPERIAL CHEMICAL INDUSTRIES PLC)<br>* claims * | 1-10 | |
| P,A | EP-A-0 548 553 (TAKEDA CHEMICAL INDUSTRIES, LTD.)<br>* claims * | 1-10 | |
| | | | TECHNICAL FIELDS SEARCHED (Int.Cl.5) |
| | | | C07D<br>A61K<br>A01N |

The present search report has been drawn up for all claims

| Place of search | Date of completion of the search | Examiner |
|---|---|---|
| THE HAGUE | 19 April 1994 | Chouly, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)